# EUROPÄISCHE PATENTSCHRIFT

(11) **EP 1 368 027 B1**
(45) Veröffentlichungstag und Bekanntmachung des Hinweises auf die Patenterteilung: **04.08.2004**
(21) Anmeldenummer: 02732330.2
(22) Anmeldetag: 15.03.2002
(51) Int. Cl.: A61K 31/405, A61P 35/00

(54) **VERWENDUNG VON TRYPTOPHAN-DERIVATEN ZUR SPEZIFISCHEN ZYTOSTATISCHEN BEHANDLUNG VON SEROTONIN-PRODUZIERENDEN TUMOREN**
USE OF TRYPTOPHAN DERIVATIVES FOR THE SPECIFIC CYTOSTATIC TREATMENT OF SEROTONIN-PRODUCING TUMORS
UTILISATION DE DERIVES DU TRYPTOPHANE POUR LE TRAITEMENT CYTOSTATIQUE SPECIFIQUE DE TUMEURS PRODUISANT DE LA SEROTONINE

(30) Priorität: 15.03.2001 DE 10112882
(43) Veröffentlichungstag der Anmeldung: 10.12.2003
(73) Patentinhaber: MAX-DELBRÜCK-CENTRUM FÜR MOLEKULARE MEDIZIN, 13125 Berlin (DE)
(72) Erfinder: WALTHER, Diego, 13187 Berlin (DE); BADER, Michael, 13125 Berlin (DE)
(74) Vertreter: Krauss, Jan
(86) Internationale Anmeldenummer: PCT/DE2002/000959
(87) Internationale Veröffentlichungsnummer: WO 2002/074309

(56) Entgegenhaltungen:
- DE-A- 2 236 876
- DE-A- 3 519 687
- US-A- 4 183 858
- US-A- 5 622 983
- US-A- 6 124 263
- DATABASE BIOSIS [Online] BIOSCIENCES INFORMATION SERVICE, PHILADELPHIA, PA, US; 1993 ZEMBOWER DAVID E ET AL: "Enantiospecific syntheses of alpha-(fluoromethyl)tryptophan analogues: Interactions with tryptophan hydroxylase an aromatic L-amino acid decarboxylase." Database accession no. PREV199395087823 XP002212214 & JOURNAL OF MEDICINAL CHEMISTRY, Bd. 36, Nr. 3, 1993, Seiten 305-313, ISSN: 0022-2623
- GILBERT JUDITH A ET AL: "Elevated aromatic-L-amino acid decarboxylase in human carcinoid tumors." BIOCHEMICAL PHARMACOLOGY, Bd. 50, Nr. 6, 1995, Seiten 845-850, XP002212211 ISSN: 0006-2952
- DATABASE BIOSIS [Online] BIOSCIENCES INFORMATION SERVICE, PHILADELPHIA, PA, US; 1982 YOFFE J R ET AL: "CHARACTERIZATION OF SEROTONIN UPTAKE IN CULTURED PHEO CHROMO CYTOMA CELLS COMPARISON WITH NOREPINEPHRINE UPTAKE" Database accession no. PREV198375002324 XP002212215 & MOLECULAR PHARMACOLOGY, Bd. 21, Nr. 2, 1982, Seiten 368-373, ISSN: 0026-895X
- DATABASE MEDLINE [Online] 1978 TUTTON P J ET AL: "Evaluation of the cytotoxicity of dihydroxytryptamines and 5-hydroxytryptamine antagonists as cytotoxic agents in dimethylhydrazine-induced adenocarcinomata." Database accession no. NLM35286 XP002212216 & CANCER CHEMOTHERAPY AND PHARMACOLOGY. GERMANY, WEST 1978, Bd. 1, Nr. 4, 1978, Seiten 209-213, ISSN: 0344-5704
- VON STUDNITZ W: "[Proceedings: Tryptophan hydroxylase inhibition as therapy for carcinoid tumors]" ZEITSCHRIFT FUR KLINISCHE CHEMIE UND KLINISCHE BIOCHEMIE. GERMANY, WEST APR 1972, Bd. 10, Nr. 4, April 1972 (1972-04), Seite 181 XP002212212 ISSN: 0044-2933
- BAUMGARTEN H G ET AL: "A developmental study of the effects of 5,7-dihydroxytryptamine on regional tryptophan hydroxylase in rat brain." PSYCHOPHARMACOLOGY COMMUNICATIONS. UNITED STATES 1975, Bd. 1, Nr. 1, 1975, Seiten 75-88, XP002212213 ISSN: 0098-616X

## Beschreibung

Die Erfindung betrifft die Verwendung von Tryptophan-Derivaten zur spezifischen zytostatischen Behandlung von Serotonin-produzierenden Tumoren, wie z.B Carcinoiden und anderen. Anwendungsgebiete der Erfindung sind die Medizin und die pharmazeutische Industrie.

Carcinoide sind ungewöhnliche neuroendokrine Tumore, die überwiegend im Gastrointestinaltrakt auftreten, und durch Transformation chromaffiner Zellen in den Lieberkühn-Krypten entstehen (Neary et al., Dis. Colon Rectum 40: 349-362, 1997), kommen aber auch in der Lunge und im Pankreas vor. Carcinoide lassen sich nur operativ entfernen, da die konventionelle adjuvante Therapie, insbesondere fortgeschrittener Serotonin-produzierender Tumore, unbefriedigend ist, weil diese Tumorzellen gegenüber herkömmlichen Zytostatika resistent sind (Neary et al., Dis. Colon Rectum 40: 349-362, 1997 ; Litvak et al., Surgery 124: 1071-1076, 1998). Daher versterben auch 3 - 18% der Patienten infolge von schneller Metastasierung des Primärtumors (Neary et al., Dis. Colon Rectum 40: 349-362, 1997; Memon und Nelson, Dis. Colon Rectum 40: 1101-1118, 1997).

Ähnlich schlechte Prognosen werden bei einem bestimmten Serotonin-produzierenden Lungentumor, dem sogenannten Small Cell Lung Carcinoma, sowie bei Mastocytomen gestellt. In allen Serotonin-produzierenden Tumoren wirkt Serotonin offenbar als autokrines Wachstumshormon, weshalb es wünschenswert ist, einerseits die Serotonin-Synthese zu reduzieren, andererseits aber auch mit spezifischen Toxinen auf die Tumorzellen einzuwirken.

Der Erfindung liegt daher die Aufgabe zugrunde, Substanzen zu suchen und bereitzustellen, die die Behandlung von Serotonin-produzierenden Tumoren ermöglichen. Diese Substanzen sollen einerseits als spezifische Zytostatika wirken, gleichzeitig sollen sie aber auch die Serotonin-Synthese als solche herabsetzen.

Die Erfindung wird gemäß den Ansprüchen realisiert. Es wurde gefunden, dass die ungiftigen Substanzen 7-Hydroxy-tryptophan und 6-Hydroxy-tryptophan (7-HTP und 6-HTP) von den Enzymen Tryptophan-Hydroxylase (TPH) und Aromatische-Aminosäure-Decarboxylase (AAAD) intrazellulär zu den hoch toxischen Substanzen 5,7- und 5,6-Dihydroxy-tryptamin (5,7- und 5,6-DHT) umgesetzt werden. Diese beiden Enzyme TPH und AAAD werden in Serotonin-produzierenden Tumorzellen außerordentlich stark exprimiert. Durch die ungleich höhere TPH-Expression in diesen Tumorzellen, im Vergleich zu normalen Serotonin-produzierenden Geweben, treten nur marginal toxische Effekte auf diese Gewebe auf.

Erfindungsgemäß werden deshalb vorzugsweise hydroxylierte Tryptophan-Derivate, die insbesondere in obengenannten malignen Zellen durch die körpereigenen Enzyme Tryptophan-Hydroxylase (TPH) und Aromatische-Aminosäure-Decarboxylase (AAAD) zu spezifischen Toxinen gegiftet werden, zur Reduzierung und/oder Hemmung der Synthese von Serotonin in Tumorzellen eingesetzt, wodurch diese Zellen spezifisch abgetötet werden. Andere Gewebe bleiben dagegen durch die Behandlung unbeeinträchtigt.

Bevorzugt werden die Tryptophan-Derivate 7-HTP, 6-HTP oder 4-HTP, sowie am aromatischen System von Tryptophan weiter substituierte Derivate eingesetzt, welche zu spezifischen Toxinen umgewandelt werden. Die Toxine 5,7-DHT und 5,6-DHT z.B. werden in der Forschung schon länger verwendet, um Läsionen von Geweben zu untersuchen, in denen der Serotonin-Transporter exprimiert wird. Dabei kommt es also zur zytotoxischen Wirkung einer Vielzahl an Serotonin-Transporter-exprimierenden Zellen, die nicht identisch mit Serotonin-produzierenden Zellen sind. Außerdem können diese Substanzen wegen ihrer hohen Oxidationsempfindlichkeit nur schwer gehandhabt werden.

Darüber hinaus wurde festgestellt, dass die Tryptophan-Hydroxylase mit hydroxylierten Tryptophan-Derivaten kompetitiv gehemmt wird, da sie in Konkurrenz mit dem eigentlichen Substrat, Tryptophan, stehen. Dies unterbindet die autokrine Wachstumsförderung des Serotonins auf diese malignen Zellen.

Erfindungsgemäß wird deshalb mit Tryptophan-Derivaten, vorzugsweise hydroxylierten Derivaten wie z.B. dem 7-HTP, 6-HTP oder 4-HTP ein effektives Zytostatikum zur Behandlung von Serotonin-produzierenden Tumoren, wie z.B Carcinoiden, bereitgestellt. Gleichzeitig hemmen die Tryptophan-Derivate gezielt die Tryptophan-Hydroxylase, wodurch ein Schutz vor der autokrinen Wachstumsförderung von Serotonin auf die Serontonin-produzierenden Tumore erzielt wird. Es können deshalb sowohl Primärtumore behandelt, als auch Metastasen zytostatisch vernichtet werden.

Die erfindungsgemäßen zytostatischen Mittel zur Chemotherapie maligner Erkrankungen auf der Basis Serotonin-produzierender Tumore sind dadurch gekennzeichnet, dass sie hydroxylierte Tryptophan-Derivate mit an sich üblichen Träger- und Hilfsstoffen enthalten, vorzugsweise die Derivate 7-HTP, 6-HTP und 4-HTP. Insbesondere werden sie zur Therapie von neuroendokrinen Tumoren, die überwiegend im Gastrotestinaltrakt, in der Lunge und im Pankreas auftreten, und bei bestimmten anderen Serotonin-produzierenden Lungentumoren, vzw. dem Small Cell Lung Carcinoma, sowie bei Mastocytomen, eingesetzt.

Die Mittel liegen zur direkten Anwendung bevorzugt in Formulierungen zur parenteralen und/oder oralen Applikation oder ggf. auch in Form von liposomalen Komplexen vor.

Die Erfindung soll nachfolgend durch Ausführungsbeispiele näher erläutert werden:

### Ausführungsbeispiele

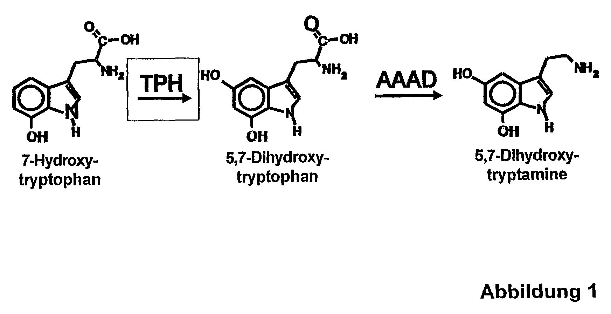

Abbildung 1. Reaktionsschema der enzymatischen Umsetzung von 7-Hydroxy-tryptophan (7-HTP) zu 5,7-Dihydroxy-tryptamin (5,7-DHT). Der geschwindigkeitsbestimmende erste Schritt wird durch das Enzym Tryptophan-Hydroxylase (TPH) vermittelt, worauf sich eine schnelle Decarboxylierung durch Aromatische-Aminosäure-Decarboxylase (AAAD) anschließt.

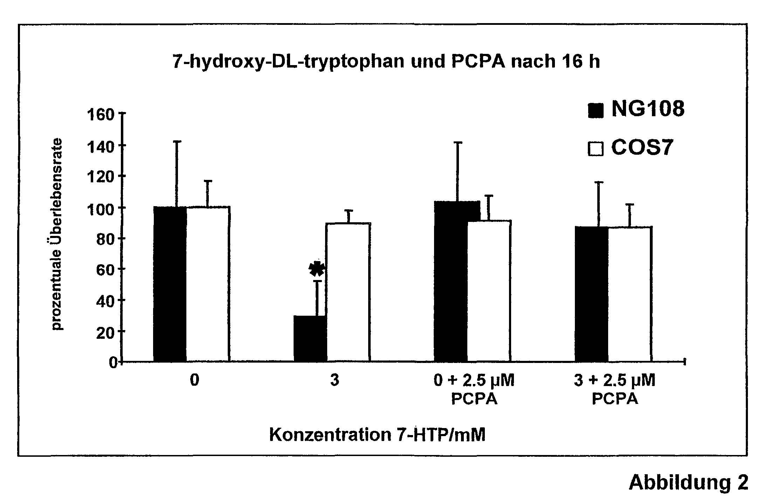

Abbildung 2. Spezifische Toxizität von 7-HTP auf die Serotonin-produzierenden Zellen NG108. Das Wachstum von COS7-Zellen, die kein Serotonin produzieren, wird dagegen nicht von 7-HTP beeinträchtigt. Mit dem spezifischen TPH-Inhibitor p-Chlorophenylalanin (PCPA) können die 7-HTP-sensitiven NG108-Zellen geschützt werden, da intrazellulär kein 5,7-DHT synthetisiert werden kann.

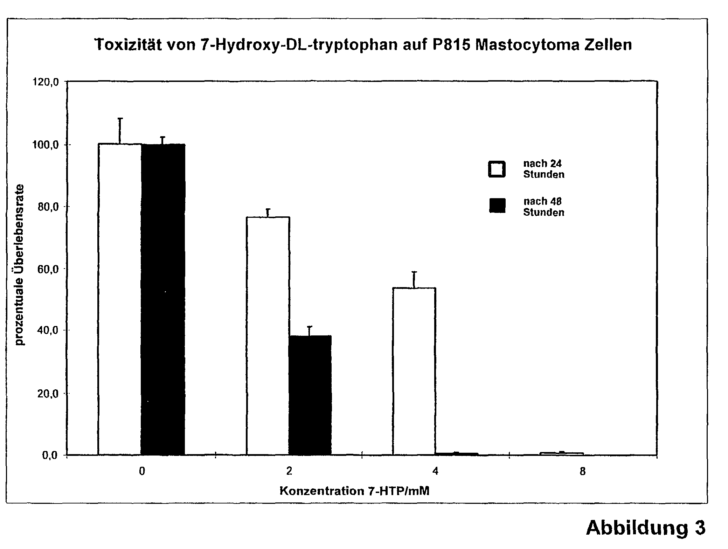

Abbildung 3. Spezifische Toxizität von 7-HTP auf die Serotonin-produzierenden Mastocytomzellen P815. Diese Tumorzellen, die sich durch hohe Resistenz gegenüber herkömmlichen Zytostatika auszeichnen, werden schnell und spezifisch durch 7-HTP abgetötet.

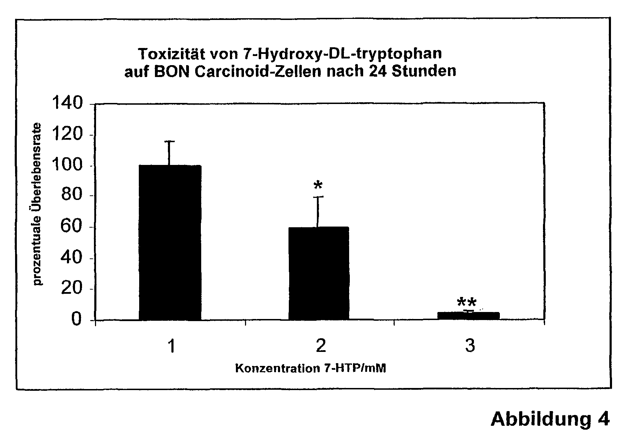

Abbildung 4. Spezifische Toxizität von 7-HTP auf die Serotonin-produzierenden humanen Carcinoidzellen BON. Diese Tumorzellen, die sich durch hohe Resistenz gegenüber herkömmlichen Zytostatika auszeichnen, werden schnell und spezifisch durch 7-HTP abgetötet.

## Patentansprüche

1. Zytostatisches Mittel zur Therapie maligner Erkrankungen, **dadurch gekennzeichnet, dass** es ein hydroxyliertes Tryptophan-Derivat, ausgewählt aus 7-Hydroxy-tryptophan (7-HTP), 6-Hydroxy-tryptophan (6-HTP) und 4-Hydroxy-tryptophan (4-HTP) und/oder am aromatischen System von Tryptophan weiter substituiertes Derivat von 7-HTP, 6-HTP oder 4-HTP mit üblichen Träger- und Hilfsstoffen enthält.

2. Verwendung eines hydroxylierten Tryptophan-Derivats, ausgewählt aus 7-Hydroxy-tryptophan (7-HTP), 6-Hydroxy-tryptophan (6-HTP) und 4-Hydroxy-tryptophan (4-HTP) zur Herstellung eines Medikaments zur Behandlung von Serotonin-produzierender Tumorzellen durch Hemmung der Tryptophan-Hydroxylase (TPH) in den Serotonin-produzierenden Tumorzellen.

3. Verwendung nach Anspruch 2, **dadurch gekennzeichnet, dass** in dem Medikament am aromatischen System von Tryptophan weiter substituierte Derivate von 7-HTP, 6-HTP oder 4-HTP eingesetzt werden.

4. Verwendung nach Anspruch 2 als Zytostatikum für Serotonin-produzierende Tumore und für die von Primärtumoren ausgelösten Metastasen.

5. Verwendung nach einem der Ansprüche 2 bis 3, **dadurch gekennzeichnet, daß** die Interaktion von TPH und Tryptophan gehemmt wird.

6. Verwendung nach einem der Ansprüche 2 bis 4, wobei die Serotonin-produzierenden Tumorzellen von neuroendokrinen Tumoren, überwiegend aus dem Gastrotestinaltrakt, der Lunge und dem Pankreas, und von bestimmten anderen Serotonin-produzierenden Lungentumoren, wie dem Small Cell Lung Carcinoma, sowie von Mastocytomen, abgeleitet sind.

## Claims

1. Cytostatic agent for the therapy of malign diseases, **characterised in that** said agent contains a hydroxylated tryptophan-derivative, selected from 7-hydroxy-tryptophan (7-HTP), 6-hydroxy-trytophan (6-HTP) and 4-hydroxy-tryptophan (4-HTP) and/or a derivative of 7-HTP, 6-HTP or 4-HTP being further substituted at the aromatic system of the tryptophan, together with common carriers and adjuvants.

2. Use of a hydroxylated tryptophan derivative, selected from 7-hydroxy-tryptophan (7-HTP), 6-hydroxy-trytophan (6-HTP) and 4-hydroxy-tryptophan (4-HTP) for the production of a medicament for the treatment of serotonin-producing tumour cells by inhibiting the tryptophan-hydroxylase (TPH) in said serotonin-producing tumour cells.

3. Use according to claim 2, **characterised in that** derivatives of 7-HTP, 6-HTP or 4-HTP being further substituted at the aromatic system of the tryptophan are used in said medicament.

4. Use according to claim 2 as cytostatic agent for serotonin-producing tumours and metastases that are released by primary tumours.

5. Use according to one of claims 2 to 3, **characterised in that** the interaction of TPH and trytophan is inhibited.

6. Use according to anyone of claims 2 to 4, wherein the serotonin-producing tumour cells are derived from neuroendocrine tumours, predominantly from the gastrointestinal tract, lung and pancreas, and from particular other serotonin-producing lung tumours, such as the small cell lung carcinoma as well as from mastocytomas.

## Revendications

1. Agent cytostatique de traitement d'affections malignes, **caractérisé en ce qu'**il contient un dérivé de tryptophane hydroxylé, sélectionné parmi le 7-hydroxy-tryptophane (7-HTP), le 6-hydroxy-tryptophane (6-HTP) et le 4-hydroxy-tryptophane (4-HTP) et/ou un dérivé de 7-HTP, 6-HTP ou 4-HTP substitué en outre au niveau du système aromatique du tryptophane avec les vecteurs ou adjuvants habituels.

2. Utilisation d'un dérivé de tryptophane hydroxylé, sélectionné parmi le 7-hydroxy-tryptophane (7-HTP), le 6-hydroxy-tryptophane (6-HTP) et le 4-hydroxy-tryptophane (4-HTP) pour produire un médicament pour le traitement de cellules tumorales produisant de la sérotonine, par inhibition de la tryptophane-hydroxylase (TPH) dans les cellules tumorales produisant la sérotonine.

3. Utilisation selon la revendication 2, **caractérisée en ce que** dans le médicament sont utilisés des dérivés du 7-HTP, du 6-HTP ou du 4-HTP substitués en outre au niveau du système aromatique du tryptophane.

4. Utilisation selon la revendication 2, comme cytostatique pour les tumeurs produisant de la sérotonine et pour les métastases déclenchées par les tumeurs primaires.

5. Utilisation selon l'une quelconque des revendications 2 à 3, **caractérisée en ce que** l'interaction de la TPH et du tryptophane est inhibée.

6. Utilisation selon l'une quelconque des revendications 2 à 4, dans laquelle les cellules tumorales produisant de la sérotonine sont dérivées de tumeurs neuro-endocriniennnes, principalement du tractus gastro-intestinal, des poumons et du pancréas, et de tumeurs pulmonaires produisant de la sérotonine, comme le carcinome pulmonaire à petites cellules ainsi que les mastocytomes.
